# EUROPEAN PATENT SPECIFICATION

(11) **EP 0 951 871 B1**
(45) Date of publication and mention of the grant of the patent: **04.07.2007**
(21) Application number: 99302529.5
(22) Date of filing: 31.03.1999
(51) Int. Cl.: A61B 17/132, A61B 19/12

(54) **A drape**
Chirurgisches Abdecktuch
Drap chirurgical

(30) Priority: 24.04.1998 GB 9808774
(43) Date of publication of application: 27.10.1999
(73) Proprietor: ArthroCare Corporation, Sunnyvale, CA 94085 (US)
(72) Inventor: Reay-Young, Clive Bruce, Atlantech Med. Dev. Ltd., Harrogate Buss. Park, Harrogate HG3 1DH (GB)
(74) Representative: Kazi, Ilya

(56) References cited:
- US-A- 4 406 281
- US-A- 4 820 279
- US-A- 5 620 001

## Description

The present invention relates to a drape and, in particular, a drape for use with a tourniquet.

In all invasive procedures carried out in operating situations it is common practice for a tourniquet to be used to prevent excessive blood loss through bleeding. The tourniquet usually consists of an inflatable cuff which is wrapped around the limb to completely encircle the limb. These cuffs usually have Velcro on them so that the cuff can stick to itself on encircling the limb. Finally a security strap is connected to prevent the cuff from unwrapping. Once in place the cuff is inflated to a desired pressure thereby imparting uniform inward pressure on the limb which constrains blood vessels reducing blood pressure on the distal side of the cuff with respect to the operation site. Common practice prior to placement of the cuff is to wrap the limb with a soft padding bandage (essentially a roll of cotton wool) to prevent pinching of the skin or abrasion from the cuff. Once the cuff is in place and inflated, the skin distal to the cuff is prepared using an alcohol based disinfecting solution to reduce risk of bacteria on the skin being transported by operative instruments onto the wound.

During preparation it is common for both the cotton wool bandage and cuff to become soaked by the solution.

The use of this bandage has a number of detrimental effects.
1. Once in contact with the alcoholic preparation solution the cotton wool absorbs and retains the solution which in some cases can cause irritation of the patients skin because of the proximity of the cotton wool and the length of time it is in place.
2. It is normal that the cotton rapidly becomes deposited within the Velcro of the cuffs thereby rendering them unusable.
3. The soaking of the cuff material itself will eventually reduce the effectiveness of the cuff.

Furthermore, cuffs have an inherent problem in that when wrapped they form a cylinder whereas the human limb arm and leg are substantially conical in shape, therefore it is common to have a problem with cuff slippage and subsequent loss of pressure.

US-A-4,406,281 discloses a cover for use with a tourniquet, the cover comprising a piece of waterproof material with a pad attached at one side.

It is an object of the present invention to address these and other problems with current tourniquets.

The invention is set out in Claim 1.

As the padded section is wholly contained within the waterproof material it is sealed away from fluid damage.

Preferably, the drape is fitted with at least one adhesive strip for encircling the limb of the user and to provide a waterproof seal between the drape and the limb of the patient. Advantageously, by locating the adhesive strip on the inside surface of the waterproof sheet, it is applied directly to the limb of the patient as soon as the drape is wrapped around the limb and thus provides a very convenient and quick seal while the limb is prepared for surgery.

Preferably, the drape is sufficiently wide to encircle a limb and the adhesive strip, preferably, extends across the width of the drape but is preferably, located partway along the length of the drape so that it may seal the distal end of the tourniquet and leave sufficient material under the tourniquet to provide a base for the tourniquet and sufficient material on the distal side of the strip to allow the drape to be folded back over the tourniquet once the latter is fitted.

Preferably, both a padded section and the adhesive strip extend across the width of the tourniquet and, preferably, the strip is located immediately adjacent the distal edge of the padded section.

Preferably, the drape is substantially rectilinear and is sufficiently wide to encircle a limb of the patient. Preferably, the padded section extends across the width of the drape and is positioned along the length of the drape in such a manner that a sufficient length of drape material remains on the distal side of the padded section so as to substantially cover a tourniquet located over the padded section, once the remaining length of the drape material is folded back thereover.

There is disclosed a method of fitting a tourniquet to a human or animal limb comprising the steps of:-
(a) wrapping the padded section of a drape in accordance with the first aspect of the present invention around the part of the same limb of the patient which is to be tourniqued;
(b) fitting and applying the tourniquet to the said padded section around the limb; and
(c) folding the drape material in the drape section over the tourniquet.

Preferably, the method in step (a) includes sticking at least one drape adhesive strip to the limb of the patient immediately adjacent the operating site at the distal end of the padded section.

There is also disclosed a method of fitting a tourniquet to a human or animal limb comprising the steps of:-
(a) wrapping a drape in accordance with the second aspect of the present invention around the part of the limb of the patient which is to be tourniqued;
(b) fitting and binding the tourniquet to the said part of the limb; and
(c) folding the drape material on the distal side of the tourniquet back over the tourniquet so that the tourniquet is in a pocket of the drape material.

Preferably, the method in step (a) includes sticking at least one drape adhesive strip to the limb of the user immediately adjacent the area over which the tourniquet is to be fitted on the distal side thereof so that a water proof seal is provided between the limb and the drape adjacent to the tourniquet, and the operating site.

The padding in the padded section may be made of any suitable material such a suitable foam material or a suitable natural fibre such as cotton wool.

Preferably, the adhesive strip is made from a hypo-allergenic material. A further adhesive strip may be provided on the underside of the drape in the area which is to be tourniqued or in the area immediately distal thereto. The adhesive strips are particularly advantageous as they prevent the tourniquet from slipping along the limb of the patient.

In addition, a further advantage is provided by the pocket which is formed by the drape in use. The pocket not only provides additional support for the tourniquet to prevent distal slippage along the limb of the user, but also provides a waterproof cover for the tourniquet to prevent damage to the tourniquet caused by the alcoholic solution generally used during surgery and also to assist sterilisation of the tourniquet during subsequent operative procedures.

A further advantage of the present invention is the use of a padded section which is completely enclosed within a waterproof jacket. Furthermore, by enclosing the padded section with a waterproof jacket, the padded section is unable to absorb the alcoholic solution, thereby avoiding damage to the skin of the patient. In this manner, the padded section is completely reusable but, more importantly, damage to the Velcro of the cuffs is thereby avoided.

Although the drape of the invention may be used in any operative procedure, it is particularly advantageous when used with a thigh tourniquet.

An embodiment of the present invention will now be described, by way of example, with reference to the accompanying drawings in which:-
Figure 1 shows a schematic plan view of a drape in accordance with the present invention; and
Figure 2 shows a schematic under plan view of a drape in accordance with the present invention.

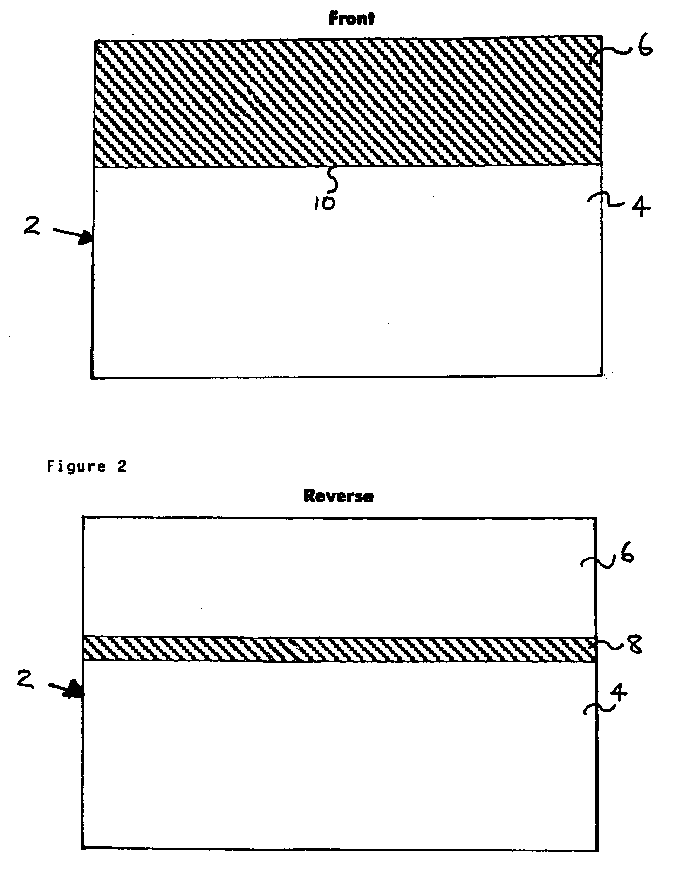

Referring to Figures 1 and 2, a waterproof drape 2 comprises a sheet of rectangular material having a drape section 4 and a padded section 6. The padded section 6 extends completely across the width of the drape and approximately one third along the length thereof. The padded section 6 comprises a sealed jacket housing a padded foam core. On the reverse side of the drape shown in figure 2, an adhesive strip 8 extends fully across the drape immediately adjacent and parallel with the distal edge 10 of the padded section 6. The adhesive strip 8 is provided with a matching strip of protected tape which may be pealed off prior to use to reveal the adhesive strip below.

The drape may be applied as follows:
1. the adhesive strip is revealed by pealing off protective tape. The drape is then wrapped around the limb, so that the adhesive strip sticks to the skin and seals the drape to the skin to provide a fluid barrier.
2. the tourniquet cuff is then wrapped around the padded section of the drape, locked and inflated.
3. the remainder of the drape which is now lying on the distal side of the cuff is folded back over the top of the cuff thus sealing the whole cuff behind a fluid barrier.
4. preparation of the skin is then undertaken.

The drape may be suitably tailored to suit particular limb tourniquets and, similarly, the length of the padded section may be appropriately dimensioned to suit the particular tourniquet.

The waterproof drape may be supplied sterile or non-sterile depending upon requirements.

Ideally, the drape will be supplied in a disposable form but washable forms may also be envisaged. The jacket which houses the padded section may be provided by stitching, heat sealing or other suitable techniques known to those skilled in the art.

All of the features disclosed in this specification (including any accompanying claims, abstract and drawings), and/or all of the steps of any method or process so disclosed, may be combined in any combination, except combinations where at least some of such features and/or steps are mutually exclusive.

The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

## Claims

1. A drape (2) for use with a tourniquet, the drape (2) comprising a piece of waterproof material (4), providing a drape section and a padded section (6) sealed within a waterproof jacket, the drape (2) arranged for encircling a patient's limb.

2. A drape for use with a tourniquet according to claim 1, wherein the drape section, in use, is adapted to be folded back over the padded section.

3. A drape according to Claim 2 arranged so that the drape may provide both a base for the tourniquet and is also adapted to be folded back thereover in use.

4. A drape for use with a tourniquet according to any preceding claim, wherein the drape is fitted with at least one adhesive strip (8) for encircling the limb of the user and to provide a waterproof seal between the drape and the limb of the patient.

5. A drape for use with a tourniquet according to any preceding claim, wherein the drape is sufficiently wide to encircle a limb.

6. A drape for use with a tourniquet according to claim 4 or 5 as dependent on claim 4, wherein the adhesive strip (8) extends substantially across the width of the drape and is located sufficiently along the length of the drape so that it may seal the limb at the distal end of the tourniquet and leave sufficient material under the tourniquet to provide a base for the tourniquet.

7. A drape for use with a tourniquet according to claim 4 or 6, wherein the adhesive strip (8) is located so that there is sufficient material on the distal side of the strip to allow the drape to be folded back over the tourniquet once the latter is fitted.

8. A drape for use with a tourniquet according to claim 4, 6 or 7, wherein the adhesive strip (8) is located immediately adjacent the distal edge of the padded section.

9. A drape according to any preceding claim wherein the padded section comprises a padded foam core.

## Patentansprüche

1. Abdecktuch (2) zur Verwendung mit einer Aderpresse, wobei das Abdecktuch (2) ein Stück aus wasserfestem Material (4) enthält, das einen Tuchabschnitt und einen gepolsterten Abschnitt (6) ergibt, der mit einem wasserfesten Mantel umschlossen ist, wobei das Abdecktuch (2) zum Umfassen eines Glieds eines Patienten vorgesehen ist.

2. Abdecktuch zur Verwendung mit einer Aderpresse nach Anspruch 1, wobei der Tuchabschnitt im Gebrauch zum Zurückfalten über den gepolsterten Abschnitt geeignet ist.

3. Abdecktuch nach Anspruch 2, so angeordnet, dass das Tuch sowohl eine Grundlage für die Aderpresse ergeben kann als auch bei der Verwendung über die Aderpresse zurückgefaltet werden kann.

4. Abdecktuch zur Verwendung mit einer Aderpresse nach einem der vorstehenden Ansprüche, wobei das Abdecktuch mit wenigstens einem Klebestreifen (8) versehen ist zum Umfassen des Glieds des Anwenders und zur Erzeugung einer wasserdichten Abdichtung zwischen dem Abdecktuch und dem Glied des Patienten.

5. Abdecktuch zur Verwendung mit einer Aderpresse nach einem der vorstehenden Ansprüche, wobei das Abdecktuch genügend breit zur Umfassung eines Glieds ist.

6. Abdecktuch zur Verwendung mit einer Aderpresse nach Anspruch 4 oder 5 zusammen mit Anspruch 4, wobei der Klebestreifen (8) sich im Wesentlichen über die Breite des Abdecktuchs erstreckt und genügend über die Länge des Abdecktuchs angebracht ist, so dass es das Glied am distalen Ende der Aderpresse abdichten kann und genügend Material unter der Aderpresse zurücklässt, um eine Grundlage für die Aderpresse zu bilden.

7. Abdecktuch zur Verwendung mit einer Aderpresse nach Anspruch 4 oder 6, wobei der Klebestreifen (8) so angeordnet ist, dass genügend Material an dem distalen Ende des Streifens ist, dass der Streifen über die Aderpresse zurückgefaltet werden kann, wenn die letztere angebracht ist.

8. Abdecktuch zur Verwendung mit einer Aderpresse nach Anspruch 4, 6 oder 7, wobei der Klebestreifen (8) unmittelbar benachbart zu dem distalen Ende des gepolsterten Abschnitts angeordnet ist.

9. Abdecktuch nach einem der vorstehenden Ansprüche, wobei der gepolsterte Abschnitt einen gepolsterten Schaumstoffkern aufweist.

## Revendications

1. Linge (2) destiné à être utilisé avec un garrot, le linge (2) comprenant une pièce de matériau imperméable (4) fournissant une section de linge et une section rembourrée étanchéifiée avec une chemise imperméable, le linge (2) étant agencé pour entourer un membre d'un patient.

2. Linge destiné à être utilisé avec un garrot selon la revendication 1, dans lequel la section de linge, en utilisation, est adaptée pour être repliée sur la section rembourrée.

3. Linge selon la revendication 2, agencé de sorte que le linge puisse fournir à la fois une base pour le garrot et soit agencé pour être replié au dessus en utilisation.

4. Linge destiné à être utilisé avec un garrot selon l'une quelconque des revendications précédentes, dans lequel le linge est muni d'au moins une bande adhésive (8) pour entourer le membre de l'utilisateur et pour fournir une étanchéité imperméable entre le linge et le membre du patient.

5. Linge destiné à être utilisé avec un garrot selon l'une quelconque des revendications précédentes, dans lequel le linge est suffisamment large pour entourer un membre.

6. Linge destiné à être utilisé avec un garrot selon la revendication 4 ou 5 lorsqu'elle est dépendante de la revendication 4, dans lequel la bande adhésive (8) s'étend essentiellement au niveau de la largeur du linge et est située suffisamment le long de la longueur du linge afin qu'elle puisse étanchéifier le membre à l'extrémité distale du garrot et laisser suffisamment de matériau sous le garrot pour fournir une base pour le garrot.

7. Linge destiné à être utilisé avec un garrot selon la revendication 4 ou 6, dans lequel la bande adhésive (8) est disposée afin qu'il y ait suffisamment de matériau sur le côté distal de la bande pour permettre au linge d'être replié sur le garrot une fois que ce dernier est adapté.

8. Linge destiné à être utilisé avec un garrot selon la revendication 4, 6 ou 7, dans lequel la bande adhésive (8) est disposée immédiatement contiguë au bord distal de la section rembourrée.

9. Linge selon l'une quelconque des revendications précédentes, dans lequel la section rembourrée comprend un noyau de mousse rembourré.
